# EUROPEAN PATENT SPECIFICATION

(11) **EP 0 391 619 B1**
(45) Date of publication and mention of the grant of the patent: **15.12.1993**
(21) Application number: 90303421.3
(22) Date of filing: 30.03.1990
(51) Int. Cl.: A61K 6/083

(54) **Dental cements**
Zahnzemente
Ciments dentaires

(30) Priority: 06.04.1989 US 333904
(43) Date of publication of application: 10.10.1990
(73) Proprietor: DEN-MAT CORPORATION, Santa Maria California 93456 (US)
(72) Inventor: Ibsen, Robert L., Santa Maria, California 93454 (US); Pacropis, Donald R., Santa Maria, California 93455 (US); Glace, William R., Orcutt, California 93455 (US)
(74) Representative: Parr, Ronald Edward R.E. Parr & Co.

(56) References cited:
- EP-A- 0 219 058
- EP-A- 0 329 268
- EP-A- 8 801 859

## Description

The present invention relates to dental cements. It is more particularly directed to light-curable dental cements based on glass ionomers.

A wide range of materials has hitherto been proposed for use as dental cements. Some of them, for example cements based on phosphoric acid and silicate cement glazes are unsatisfactory inter alia because they are incompatible with dental pulp. Others, for example cements based on polycarboxylic acids and metal oxides, although compatible with dental pulp, are unsatisfactory beause they are brittle.

Eurpean Patent Specification 0 219 058 discloses a dental cement comprising:
(a) polymerizable unsaturated monomer;
(b) finely divided reactive filler; and
(c) a curing agent.

The polymerizable unsaturated monomer is a resin binder such as bis-GMA that reacts with reactive fillers to make the dental cement adhere to dental tissue.

Ionomer cements are a recent advance in the practice of dentistry. Some of the better of such compositions are described in US Patent 4 738 722 to lbsen,Glace and Pacropis, assigned to the applicant hereof. These cements have excellent adhesion and give minimal pulpal trauma, but tend to cure rather slowly, a disadvantage in many dental procedures.

The cements of the present invention have the advantages of the cements described in US 4 738 722; in addition they have enhanced physical strength and bonding, but most important they have the ability to cure very quickly on exposure to light.

Accordingly, the present invention provides a dental cement system whose ingredients are packaged in at least two separate package parts, the ingredients in each package part being selected so that undesirable reaction between ingredients in each package part is avoided,
characterised in that said ingredients comprise:
1. a dental cement glass;
2. barium tungstate;
3. zinc oxide and/or titanium dioxide;
4. NTG-GMA and/or an alkali metal salt thereof;
5. ethyl 4-dimethylamino benzoate;
6. an ethoxylated bisphenol A dimethacrylate;
7. 2-hydroxyethyl methacrylate;
8. 2,3-bornanedione;
9. a butylated hydroxytoluene;
10. polymer selected from polyacrylic acid, polymaleic acid, polyitaconic acid and copolymers of two or more of acrylic acid, maleic acid and itaconic acid;
11. benzoyl peroxide; and
12. PMDM.

Details of preferred ingredients (or components) 1 to 12 referred to above are as follows:
1. Glass. Any of the types customarily used in dental cements can be employed, but a preferred type is that from which fluoride ions are leached. A glass which is especially preferred is an alumina fluoride silica glass made by Specialty Glass Company of Oldsmar, Florida, and designated SP912-1 Glass.
2. barium tungstate
3. zinc oxide or titanium dioxide
4. NTG-GMA and/or the sodium or other alkali metal salt of NTG-GMA. This material is made by the Esschem Corporation and believed to have the structure. 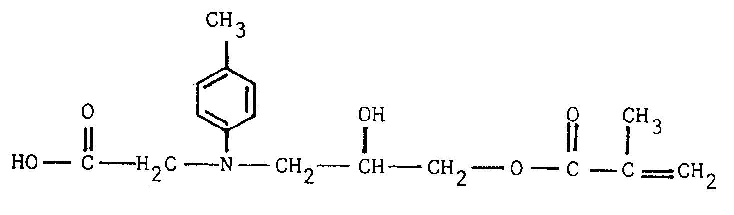
5. ethyl 4-dimethylamino benzoate.
6. ethoxylated bisphenol A dimethacrylate.
7. 2-hydroxyethyl methacrylate.
8. 2,3-bornanedione.
9. butylated hydroxytolune.
10. polyacrylic acid, polymaleic acid, polyitaconic acid,or a copolymer of acrylic acid, maleic acid or itaconic acid. These polymers should preferably have molecular weights M_{w} of 3000 - 250,000, and can be easily prepared by conventional techniques. This component preferably contains about 5% of d-tartaric acid.
11. benzoyl peroxide.
12. PMDM - made by Esschem Corporation and believed to be a pyromellitic dianhydride extended with methyl methacrylate.

The concentrations of the ingredients will, of course, vary according to the viscosities desired. Thus, for example the dental cement system can be in one of two forms depending on the viscosity needed. The first is a powder/liquid whose viscosity can be varied according to the powder/liquid ratio used when the powder and liquid are mixed. The second is a paste/paste whose viscosity can be selected when the pastes are mixed.

The cements of the present invention are conveniently in a two-part system, each part of which can be packaged separately for storage and sale. These parts are mixed together just before use, thus ensuring a fresh and vigorous cement.

A first two-part system of the invention comprises parts (a) and (b) in which
part (a) comprises the following ingredients:
1. a dental cement glass
2. barium tungstate
3. zinc oxide and/or titanium dioxide
4. NTG-GMA and/or an alkali metal salt thereof
5. ethyl 4-dimethylamino benzoate; and
   part (b) comprises the following ingredients:
6. an ethoxylated bisphenol A dimethacrylate
7. 2-hydroxyethyl methacrylate
8. PMDM
9. a polymer selected from polyacrylic acid, polymaleic acid, polyitaconic acid and copolymers of two or more of acrylic acid,maleic acid and itaconic acid.
10. 2,3-bornanedione
11. a butylated hydroxytoluene
12. benzoyl peroxide
13. tartaric acid,

wherein parts (a) and (b) are stored separately in said system so as to prevent undesirable interaction between them.

In a preferred form of said first two-part system part (a) is a powder and part (b) a liquid. Details of preferred ingredients of the powder and liquid are as follows:

### Powder

glass 65-90% by weight, preferably about 82%
barium tungstate 0-15%, preferably about 7%
zinc oxide 0 - 10% preferably about 2%
equilibrium mixture of Na NTG-GMA with NTG-GMA 5- 10%, preferably about 8%
ethyl 4-dimethylamino benzoate 0 - 2%, preferably about 1%

### Liquid

ethoxylated bisphenol A dimethacrylate - 50-80% preferably about 73.71%
2-hydroxyethyl methacrylate - 10-25%, preferably about 18%
PMDM 2.5 - 17%, preferably about 5%
polyacrylic acid (or other polymer) - 1-5%, preferably about 3.1%
2,3-bornanedione - 0.05- .25% preferably about 0.14%
butylated hydroxytoluene - 0.01 - .08%, preferably about 0.03%
benzoyl peroxide - 0.005 - .05% preferably about 0.02%
tartaric acid 0.10 - 1%, preferably about 0.12%

The powder and the liquid can be made, for example by mixing the listed components, in any order, under ambient conditions. To prepare the system for use, the powder and the liquid are mixed at a preferred powder/liquid weight ratio of 2/1-1/1, as determined by the viscosity desired of the cement product.

A second two-part system of the invention comprises parts (a) and (b) in which
part (a) comprises the following ingredients:
1. a dental cement glass
2. 2-hydroxyethyl methacrylate
3. an ethoxylated bisphenol A dimethacrylate
4. NTG-GMA and/or an alkali metal salt thereof
5. zinc oxide
6. barium tungstate
7. ethyl 4-dimethylamino benzoate;
   and part (b) comprises the following ingredients:
8. a dental cement glass
9. 2-hydroxyethyl methacrylate
10. an ethoxylated bisphenol A dimethacrylate
11. 2,3-bornanedione
12. a butylated hydroxytoluene
13. benzoyl peroxide
14. a polymer selected from polyacrylic acid, polymaleic acid, polyitaconic acid and copolymers of two or more of acrylic acid, maleic acid and itaconic acid
15. PMDM,
   wherein parts (a) and (b) are stored separately in said system so as to prevent undesirable interaction betwen them.

In a preferred form of said second two-part system part (a) and part (b) are pastes. Details of preferred ingredients of the two pastes (referred to below as Paste A and Paste B) are as follows:

### Paste A

glass 18 - 60%, preferably about 48%
2-hydroxyethyl methacrylate 5-15%, preferably about 7% ethoxylated bisphenol A dimethacrylate 20-50%,
preferably about 27%
equilibrium mixture of Na NTG-GMA with NTG-GMA 5-15%,
preferably about 8%
zinc oxide 0 - 15%, preferably about 2%
barium tungstate 0 - 15%, preferably about 7%
ethyl 4-dimethylamino benzoate 0 - 2%, preferably about 1%

### Paste B

glass 25-65%, preferably about 57%
2-hydroxyethyl methacrylate 5-15%, preferably about 7%
ethoxylated bisphenol A dimethacrylate 20-50%, preferably about 28%
2,3-bornanedione 0.05-.30%,preferably about 0.1%
butylated hydroxytoluene 0.005-.10%,preferably about 0.05%
benzoyl peroxide 0.005-.05%,preferably about 0.01%
polyacrylic acid (or other polymer) 1-5% preferably about 3%
PMDM 2.5-17%, preferably about 5%

The pastes can be made, for example, by simply mixing the listed components, in any order, under ambient conditions. To prepare the system for use, the pastes are mixed at a preferred paste A/paste B weight ratio of about 1/1.

With particular reference to the powder/liquid and paste/paste systems described above their mode of use can be as follows.

The dentist uses the system by combining the powder and liquid or the two pastes in the ratios desired, and then mixing them. The resulting cement is then applied to a tooth as needed, according to recognized principles of dental practice. The cement will self-cure in about 20-30 minutes, but cures instantly on exposure to light. Light having a wave length of about 480nM at an intensity of about 5000 foot-candles is preferred. An exposure of about 30 seconds is sufficient to cure the cement in most applications.

The glass ingredient of the dental cement systems of the present invention can be, for example, an aluminium fluoride silica glass, an aluminium fluoride silicate glass, an alumina fluoride silica glass or an alumina fluoride silicate glass. Examples of such glasses are disclosed in our U.S. Patent 4 738 722.

## Claims

1. A dental cement system whose ingredients are packaged in at least two separate package parts, the ingredients in each package part being selected so that undesirable reaction between ingredients in each package part is avoided,
characterised in that said ingredients comprise:
1. a dental cement glass;
2. barium tungstate;
3. zinc oxide and/or titanium dioxide;
4. NTG-GMA and/or an alkali metal salt thereof;
5. ethyl 4-dimethylamino benzoate;
6. an ethoxylated bisphenol A dimethacrylate;
7. 2-hydroxyethyl methacrylate;
8. 2,3-bornanedione;
9. a butylated hydroxytoluene;
10. polymer selected from polyacrylic acid,polymaleic acid, polyitaconic acid and copolymers of two or more of acrylic acid, maleic acid and itaconic acid;
11. benzoyl peroxide; and
12. PMDM.

2. A dental cement system according to Claim 1, which also contains tartaric acid in the package part containing ingredient 10.

3. A dental cement system according to Claim 1 or 2, in which the glass is a glass from which, in use, fluoride ions are leached.

4. A dental cement system according to Claim 1, 2 or 3, in which the glass is an alumina fluoride silicate glass.

5. A dental cement system according to any of the preceding claims, in which said polymer is polyacrylic acid.

6. A dental cement system according to Claim 1, comprising two parts (a) and (b),
characterised in that part (a) comprises the following ingredients:
1. a dental cement glass
2. barium tungstate
3. zinc oxide and/or titanium dioxide
4. NTG-GMA and/or an alkali metal salt thereof
5. ethyl 4-dimethylamino benzoate
and part (b) comprises the following ingredients:
6. an ethoxylated bisphenol A dimethacrylate
7. 2-hydroxyethyl methacrylate
8. PMDM
9. a polymer selected from polyacrylic acid, polymaleic acid, polyitaconic acid and copolymers of two or more of acrylic acid, maleic acid and itaconic acid
10. 2,3-bornanedione
11. a butylated hydroxytoluene
12. benzoyl peroxide
13. tartaric acid,
wherein parts (a) and (b) are stored separately in said system so as to prevent undesirable interaction between them.

7. A dental cement system according to Claim 1, comprising two parts (a) and (b),
characterised in that
part (a) comprises the following ingredients:
1. a dental cement glass
2. 2-hydroxyethyl methacrylate
3. an ethoxylated bisphenol A dimethacrylate
4. NTG-GMA and/or an alkali metal salt thereof
5. zinc oxide
6. barium tungstate
7. ethyl 4-dimethylamino benzoate;
and part (b) comprises the following ingredients:
8. a dental cement glass
9. 2-hydroxyethyl methacrylate
10. an ethoxylated bisphenol A dimethacrylate
11. 2,3-bornanedione
12. a butylated hydroxytoluene
13. benzoyl peroxide
14. a polymer selected from polyacrylic acid, polymaleic acid, polyitaconic acid and copolymers of two or more of acrylic acid, maleic acid and itaconic acid
15. PMDM
wherein parts (a) and (b) are stored separately
in said system so as to prevent undesirable interaction between them.

8. A dental cement system according to Claim 1, characterised in that the system is a two-part system composed of:
(a) a powder comprising:
1. alumina fluoride silicate glass
2. barium tungstate
3. zinc oxide
4. an equilibrium mixture of Na NTG-GMA with NTG-GMA
5. ethyl 4-dimethylamino benzoate; and,
(b) a liquid comprising:
6. ethoxylated bisphenol A dimethacrylate
7. 2-hydroxyethyl methacrylate
8. PMDM
9. polyacrylic acid
10. 2,3-bornanedione
11. butylated hydroxytoluene
12. benzoyl peroxide
13. tartaric acid
wherein parts (a) and (b) are stored separately in said system so as to prevent undesirable interaction between them.

9. A dental cement system according to Claim 8, in which the components are present in the following concentrations by weight, the total of said concentrations being 100%.
1. 65-90%
2. 0-15%
3. 0.0-10%
4. 5-10%
5. 0.0-2%
6. 50-80%
7. 10-25%
8. 2.5-17%
9. 1-5%
10. .05-.25%
11. .01-.08%
12. .005-.05%
13. 0.10-1%

10. A dental cement system according to Claim 8, in which the components are present in the following concentrations by weight, the total of said concentrations being 100%.
1. 82%
2. 7%
3. 2%
4. 8%
5. 1%
6. 73.71%
7. 18%
8. 5%
9. 3.1%
10. 0.14%
11. 0.03%
12. 0.02%
13. 0.12%

11. A dental cement system according to Claim 6,8,9 or 10, in which parts (a) and (b) are present in said system in amounts by weight in a ratio in the range from 2:1 to 1:1.

12. A dental cement system according to Claim 1, characterised in that the system is a two-part system composed of:
a a paste comprising:
1. alumina fluoride silica glass
2. 2-hydroxyethyl methacrylate
3. ethoxylated bisphenol A dimethacrylate
4. an equilibrium mixture of Na NTG-GMA with NTG-GMA
5. zinc oxide
6. barium tungstate
7. ethyl 4-dimethylamino benzoate; and,
b a paste comprising:
8. alumina fluoride silica glass
9. 2-hydroxyethyl methacrylate
10. ethoxylated bisphenol A dimethacrylate
11. 2,3-bornanediol
12. butylated hydroxytoluene
13. benzoyl peroxide
14. polyacrylic acid
15. PMDM
wherein parts (a) and (b) are stored separately in said system so as to prevent undesirable interaction between them.

13. A dental cement system according to Claim 12, in which the components are present at the following concentrations by weight, the total of said concentrations being 100%:
1. 18-60%
2. 5-15%
3. 20-50%
4. 5-15%
5. 0.0-15%
6. 0-15%
7. 0.0-2%
8. 25-65%
9. 5-15%
10. 20-50%
11. 0.05-0.30%
12. .005-.10%
13. .005-0.05%
14. 1-5%
15. 2.5-17%

14. A dental cement system according to Claim 12, in which the components are present in the following concentrations by weight , the total of said concentrations being 100%:
1. 48%
2. 7%
3. 27%
4. 8%
5. 2%
6. 7%
7. 1%
8. 57%
9. 7%
10. 28%
11. 0.1%
12. .05%
13. .01%
14. 3%
15. 5%

15. A dental cement system according to Claim 7, 12, 13, or 14, in which parts (a) and (b) are present in said system in amounts by weight in a ratio of about 1:1.

## Patentansprüche

1. Dentalzementsystem, dessen Bestandteile in wenigstens zwei getrennten Verpackungsteilen verpackt sind, wobei die Bestandteile in jedem Verpackungsteil derart ausgewählt sind, daß unerwünschte Reaktionen zwischen Bestandteilen in jedem Packungsteil ausgeschlossen sind,
dadurch gekennzeichnet, daß die Bestandteile enthalten:
1. ein Dentalzementglas;
2. Bariumwolframat;
3. Zinkoxid und/oder Titandioxid;
4. NTG-GMA und/oder ein Alkali-Metallsalz davon;
5. Ethyl-4-Dimethylamino-Benzoat;
6. ein ethoxyliertes Bisphenol-A-Dimethacrylat;
7. 2-Hydroxyethyl-Methacrylat;
8. 2,3-Bornandion;
9. ein butyliertes Hydroxytoluol;
10. Polymer, ausgewählt aus Polyacrylsäure, Polymaleinsäure, Polyitaconsäure und Copolymeren von zwei oder mehreren von Acrylsäure, Maleinsäure und Itaconsäure;
11. Benzoyl-Peroxid;
12. PMDM.

2. Dentalzementsystem nach Anspruch 1, welches außerdem in dem den Bestandteil 10 enthaltenen Verpackungsteil Weinsäure enthält.

3. Dentalzementsystem nach Anspruch 1 oder 2, bei welchem das Glas ein Glas ist, von dem bei Verwendung Fluoridionen extrahiert werden.

4. Dentalzementsystem nach Anspruch 1, 2 oder 3, bei welchem das Glas ein Tonerde-Flourid-Kieselerdeglas ist.

5. Dentalzementsystem nach einem der vorhergehenden Ansprüche, bei welchem das Polymer Polyacrylsäure ist.

6. Dentalzementsystem nach Anspruch 1, welches zwei Teile (a) und (b) enthält,
dadurch gekennzeichnet, daß Teil (a) die folgenden Bestandteile aufweist:
1. ein Dentalzementglas
2. Bariumwolframat
3. Zinkoxid und/oder Titaniumdioxid
4. NTG-GMA und/oder ein Alkalimetallsalz davon
5. Ethyl-4-Dimethylamino-Benzoat
und Teil (b) die folgenden Bestandteile aufweist:
6. ein ethoxyliertes Bisphenol-A-Dimethacrylat
7. 2-Hydroxyethyl-Methacrylat
8. PMDM
9. ein Polymer, ausgewählt aus Polyacrylsäure, Polymaleinsäure, Polyitakonsäure und Copolymeren von zwei oder mehr von Acrylsäure, Maleinsäure und Itakonsäure
10. 2,3-Bornandion
11. ein butyliertes Hydroxytoluol
12. Benzoylperoxid
13. Weinsäure,
wobei die Teile (a) und (b) in dem System getrennt gelagert werden, um unerwünschte Wechselwirkungen zwischen ihnen zu verhindern.

7. Dentalzementsystem nach Anspruch 1, welches zwei Teile (a) und (b) aufweist,
dadurch gekennzeichnet, daß
Teil (a) die folgenden Bestandteile aufweist:
1. ein Dentalzementglas
2. 2-Hydroxyethyl-Methacrylat
3. ein ethoxyliertes Bisphenol-A-Dimethacrylat
4. NTG-GMA und/oder ein Alkalimetallsalz davon
5. Zinkoxid
6. Bariumwolframat
7. Ethyl-4-Dimethylamino-Benzoat
und Teil (b) die folgenden Bestandteile aufweist:
8. ein Dentalzementglas
9. 2-Hydroxyethyl-Methacrylat
10. ein ethoxyliertes Bisphenol-A-Dimethacrylat
11. 2,3-Bornandion
12. ein butyliertes Hydroxytoluol
13. Benzoylperoxid
14. ein Polymer, ausgewählt aus Polyacrylsäure, Polymaleinsäure, Polyitakonsäure und Copolymeren von zwei oder mehr von Acrylsäure, Maleinsäure und Itakonsäure
15. PMDM
wobei die Teile (a) und (b) in dem System getrennt gelagert werden, um unerwünschte Wechselwirkungen zwischen ihnen zu verhindern.

8. Dentalzementsystem nach Anspruch 1, dadurch gekennzeichnet, daß das System ein zweiteiliges System ist, zusammengesetzt aus:
(a) einem Pulver enthaltend:
1. Tonerde-Flourid-Kieselerdeglas
2. Bariumwolframat
3. Zinkoxid
4. einer Gleichgewichtsmischung aus Na NTG-GMA mit NTG-GMA
5. Ethyl-4-Dimethylamino-Benzoat;
(b) einer Flüssigkeit enthaltend:
6. ethoxyliertes Bisphenol-A-Dimethacrylat
7. 2-Hydroxyethyl-Methacrylat
8. PMDM
9. Polyacrylsäure
10. 2,3-Bornandion
11. butyliertes Hydroxytoluol
12. Benzoylperoxid
13. Weinsäure,
wobei die Teile (a) und (b) in dem System getrennt voneinander gelagert werden, um unverwünschte Wechselwirkungen zwischen ihnen zu verhindern.

9. Dentalzementsystem nach Anspruch 8, bei welchem die Komponenten in den folgenden Gewichtskonzentrationen vorliegen, wobei die Summe der Konzentrationen 100% ist:
1. 65-90%
2. 0-15%
3. 0,0-10%
4. 5-10%
5. 0,0-2%
6. 50-80%
7. 10-25%
8. 2,5-17%
9. 1-5%
10. 0,05-0,25%
11. 0,01-0,08%
12. 0,005-0,05%
13. 0,10 -1% .

10. Dentalzementsystem nach Anspruch 8, bei welchem die Komponenten in den folgenden vorliegen, wobei die Summe der Konzentrationen 100% ist:
1. 82%
2. 7%
3. 2%
4. 8%
5. 1%
6. 73,71%
7. 18%
8. 5%
9. 3,1%
10. 0,14%
11. 0,03%
12. 0,02%
13. 0,12%.

11. Dentalzementsystem nach Anspruch 6, 8, 9 oder 10, bei dem die Teile (a) und (b) in dem System in Gewichtsmengen in einem Verhältnis im Bereich von 2:1 bis 1:1 vorliegen.

12. Dentalzementsystem nach Anspruch 1, dadurch gekennzeichnet, daß das System ein zweiteiliges System ist, zusammengesetzt aus:
a einer Paste enthaltend:
1. Tonerde-Fluorid-Kieselerdeglas
2. 2-Hydroxyethyl-Methacrylat
3. ethoxyliertes Bisphenol-A-Dimethacrylat
4. eine Gleichgewichtsmischung aus Na NTG-GMA mit NTG-GMA
5. Zinkoxid
6. Bariumwolframat
7. Ethyl-4-Dimethylaminobenzoat; und
b einer Paste enthaltend:
8. Tonerde-Fluorid-Kieselerdeglas
9. 2-Hydroxyethyl-Methacrylat
10. ethoxyliertem Bisphenol-A-Dimethacrylat
11. 2,3-Bornandiol
12. butyliertes Hydroxytoluol
13. Benzoylperoxid
14. Polyacrylsäure
15. PMDM,
wobei die Teile (a) und (b) in dem System getrennt voneinander gelagert werden, um unerwünschte Wechselwirkungen zwischen ihnen zu verhindern.

13. Dentalzementsystem nach Anspruch 12, bei welchem die Komponenten in folgenden Gewichtskonzentrationen vorliegen, wobei die Summe der Konzentrationen 100% beträgt:
1. 18 - 60%
2. 5 - 15%
3. 20 - 50%
4. 5 - 15%
5. 0,0 - 15%
6. 0 - 15%
7. 0,0 - 2%
8. 25 - 65%
9. 5 - 15%
10. 20 - 50%
11. 0,05 - 0,30%
12. 0,005- 0,10%
13. 0,005- 0,05%
14. 1 - 5%
15. 2,5 - 17%.

14. Dentalzementsystem nach Anspruch 12, bei welchem die Komponenten in den folgenden Gewichtskonzentrationen vorliegen, wobei die Summe der Konzentrationen 100% beträgt:
1. 48%
2. 7%
3. 27%
4. 8%
5. 2%
6. 7%
7. 1%
8. 57%
9. 7%
10. 28%
11. 0,1%
12. 0,05%
13. 0,01%
14. 3%
15. 5%.

15. Dentalzementsystem nach Anspruch 7, 12, 13 oder 14, bei welchem die Teile (a) und (b) in dem System in Gewichtsmengen in einem Verhältnis von etwa 1:1 vorliegen.

## Revendications

1. Système d'amalgame dentaire dont les éléments sont conditionnés dans au moins deux parties de conditionnement séparées, les éléments dans chaque partie du conditionnement étant choisis de façon à ce qu'une réaction indésirable entre les éléments dans chaque partie du conditionnement soit évitée,
caractérisé en ce que lesdits éléments comprennent:
1. un verre d'amalgame dentaire;
2. tungstate de baryum;
3. oxyde de zinc et/ou dioxyde de titane;
4. NTG-GMA et/ou un sel de métal alcalin de celui-ci;
5. 4-diméthylaminobenzoate d'éthyle;
6. un diméthylacrylate A bisphénol éthoxylé;
7. méthacrylate de 2-hydroxyéthyle;
8. 2,3-bornanedione;
9. un hydroxytoluène butylé;
10. polymère choisi parmi acide polyacrylique, acide polymaléique, acide polyitaconique et copolymères de deux ou plus d' acide acrylique, d'acide maléique et d'acide itaconique;
11. peroxyde de benzoyle; et
12. PMDM

2. Système d'amalgame dentaire selon la Revendication 1, qui contient aussi l'acide tartrique dans la partie du conditionnement contenant l'élément 10.

3. Système d'amalgame dentaire selon la Revendication 1 ou 2, dans lequel le verre est un verre à partir duquel, pendant l'utilisation, des ions fluorure sont libérés.

4. Système d'amalgame dentaire selon la Revendication 1, 2 ou 3, dans lequel le verre est un verre de silicate de flurore d'aluminium.

5. Système d'amalgame dentaire selon l'une quelconque des revendications précédentes , dans lequel ledit polymère est l'acide polyacrylique.

6. Système d'amalgame dentaire selon la Revendication 1, comprenant deux parties (a) et (b),
caractérisé en ce que la partie (a) comprend les éléments suivants:
1. un verre d'amalgame dentaire;
2. tungstate de baryum;
3. oxyde de zinc et/ou dioxyde de titane;
4. NTG-GMA et/ou un sel de métal alcalin de celui-ci;
5. 4-diméthylaminobenzoate d'éthyle;
et la partie (b) comprend les éléments suivants:
6. un diméthylacrylate A bisphénol éthoxylé;
7. méthacrylate de 2-hydroxyéthyle;
8. PMDM
9. Un polymère choisi parmi acide polyacrylique, acide polymaléique, acide polyitaconique et copolymères de deux ou plus d' acide acrylique, d'acide maléique et d'acide itaconique
10. 2,3-bornanedione
11. un hydroxytoluène butylé
12. peroxyde de benzoyle
13. acide tartrique
dans lequel les parties (a) et (b) sont stockées séparément dans ledit système de façon à prévenir une interaction indésirable entre elles.

7. Système d'amalgame dentaire selon la revendication 1, comprenant deux parties (a) et (b), caractérisé en ce que
la partie (a) comprend les éléments suivants:
1. un verre d'amalgame dentaire
2. méthacrylate de 2-hydroxyéthyle
3. un diméthylacrylate A bisphénol éthoxylé;
4. NTG-GMA et/ou un sel de métal alcalin de celui-ci
5. oxyde de zinc
6. tungstate de baryum
7. 4-diméthylaminobenzoate d'éthyle;
et la partie (b) comprend les éléments suivants
8. un verre d'amalgame dentaire
9. méthacrylate de 2-hydroxyéthyle
10. un diméthylacrylate A bisphénol éthoxylé
11. 2,3-bornanedione
12. un hydroxytoluène butylé
13. peroxyde de benzoyle
14. Un polymère choisi parmi acide polyacrylique, acide polymaléique, acide polyitaconique et copolymères de deux ou plus d' acide acrylique, d'acide maléique et d'acide itaconique
15. PMDM
dans lequel les parties (a) et (b) sont stockées séparément dans ledit système de façon à prévenir une interaction indésirable entre elles.

8. Système d'amalgame dentaire selon la Revendication 1, caractérisé en ce que le système est un système en deux parties composé de:
(a) une poudre comprenant:
1. un verre de silicate de fluorure d'aluminium
2. tungstate de baryum
3. oxyde de zinc
4. un mélange à l'équilibre de sel de Na de NTG-GMA avec NTG-GMA
5. 4-diméthylaminobenzoate d'éthyle; et,
(b) un liquide comprenant:
6. diméthylacrylate A bisphénol éthoxylé
7. méthacrylate de 2-hydroxyéthyle
8. PMDM
9. acide polyacrylique
10. 2,3-bornanedione
11. hydroxytoluène butylé
12. peroxyde de benzoyle
13. acide tartrique
dans lequel les parties (a) et (b) sont stockées séparément dans ledit système de façon à prévenir une interaction indésirable entre elles.

9. Système d'amalgame dentaire selon la Revendication 8, dans lequel les composants sont présents avec les concentrations suivantes en poids, le total desdites concentrations étant de 100 %.
1. 65-90 %
2. 0-15 %
3. 0,0-10 %
4. 5-10 %
5. 0,0-2%
6. 50-80 %
7. 10-25 %
8. 2,5-17 %
9. 1-5 %
10. 0,05-0,25 %
11. 0,01-0,08 %
12 0,005-0,05 %
13. 0,10-1 %

10. Système d'amalgame dentaire selon la Revendication 8, dans lequel les composants sont présents avec les concentrations suivantes en poids, le total desdites concentrations étant de 100 %.
1. 82 %
2. 7 %
3. 2 %
4. 8 %
5. 1 %
6. 73,71 %
7. 18 %
8. 5 %
9. 3,1 %
10. 0,14 %
11. 0,03 %
12. 0,02 %
13. 0,12 %

11. Système d'amalgame dentaire selon la Revendication 6, 8, 9 ou 10 dans lequel les parties (a) et (b) sont présentes dans ledit système avec des quantités en poids dans un rapport dans la gamme de 2:1 à 1:1.

12. Système d'amalgame dentaire selon la Revendication 1, caractérisé en ce que le système est un système à deux parties composées de :
(a) une pâte comprenant:
1. un verre de silicate de fluorure d'aluminium
2. méthacrylate de 2-hydroxyéthyle
3. diméthylacrylate A bisphénol éthoxylé
4. un mélange à l'équilibre de sel de Na de NTG-GMA et de NTG-GMA
5. oxyde de zinc
6. tungstate de baryum
7. 4-diméthylaminobenzoate d'éthyle; et
(b) pâte comprenant:
8. un verre de silicate de fluorure d'aluminium
9. méthacrylate de 2-hydroxyéthyle
10. diméthylacrylate A bisphénol éthoxylé
11. 2,3-bornanediol
12. hydroxytoluène butylé
13. peroxyde de benzoyle
14. acide polyacrylique
15. PMDM
dans lequel les parties (a) et (b) sont stockées séparément dans ledit système de façon à prévenir use interaction indésirable entre elles.

13. Système d'amalgame dentaire selon la Revendication 12, dans lequel les composants sont présents avec les concentrations suivantes en poids, le total desdites concentrations étant de 100 %.
1. 18-60 %
2. 5-15 %
3. 20-50 %
4. 5-15 %
5. 0,0-15 %
6. 0-15 %
7. 0,0-2 %
8. 25-65 %
9. 5-15 %
10. 20-50 %
11. 0,05-0,30 %
12 0,005-0,10 %
13. 0,005-0,05 %
14. 1-5 %
15. 2,5-17 %

14. Système d'amalgame dentaire selon la Revendication 12, dans lequel les composants sont présents avec les concentrations suivantes en poids, le total desdites concentrations étant de 100 %.
1. 48 %
2. 7 %
3. 27 %
4. 8 %
5. 2 %
6. 7 %
7. 1 %
8. 57 %
9. 7 %
10. 28 %
11. 0,1 %
12. 0,05 %
13. 0,01 %
14. 3 %
15. 5 %

15. Système d'amalgame dentaire selon la Revendication 7, 12, 13 ou 14 dans lequel les parties (a) et (b) sont présentes dans ledit système avec des quantités en poids dans un rapport d'environ 1:1.
